(19) Europäisches Patentamt
European Patent Office
Office européen des brevets

(11) Publication number: **0 144 118**
**B1**

(12) **EUROPEAN PATENT SPECIFICATION**

(45) Date of publication of patent specification: **15.07.87**

(51) Int. Cl.⁴: **C 07 C 69/68,** C 07 C 59/08, C 07 C 67/38, C 07 C 51/00

(21) Application number: **84305611.0**

(22) Date of filing: **17.08.84**

(54) **Alkoxycarbonylation or carbonylation with CO and organic hydroxyl compound.**

(30) Priority: **29.08.83 US 527051**

(43) Date of publication of application:
**12.06.85 Bulletin 85/24**

(45) Publication of the grant of the patent:
**15.07.87 Bulletin 87/29**

(84) Designated Contracting States:
**BE CH DE FR GB IT LI NL**

(56) References cited:
**DE-A-2 623 673**
**DE-A-3 210 617**

**The file contains technical information submitted after the application was filed and not included in this specification**

(73) Proprietor: **THE STANDARD OIL COMPANY**
**Midland Building**
**Cleveland, Ohio 44115 (US)**

(72) Inventor: **Cesa, Mark Clark**
**4617 Birchwold Road**
**South Euclid, OH 44121 (US)**
Inventor: **Burrington, James David**
**23860 Harms Road**
**Richmond Heights, OH 44143 (US)**

(74) Representative: **Smith, Sydney et al**
**Elkington and Fife High Holborn House 52/54**
**High Holborn**
**London WC1V 6SH (GB)**

**EP 0 144 118 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention relates to a new reaction wherein an enol acylate is reacted with CO and a hydroxyl compound to add an esterified carboxy group to the vinyl enol carbon atom of the enol acylate. The enol acylate may be vinyl acetate in a now preferred embodiment of the process, and the esterified product can be hydrolyzed to produce lactic acid.

More specifically, according to the present invention we provide a process comprising intimately admixing and reacting an enol acylate,

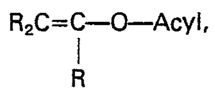

with CO and a hydroxyl compound

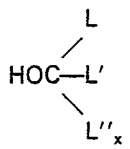

which is free of ethylenic or acetylenic unsaturation to produce an acyloxy ester compound,

(A) 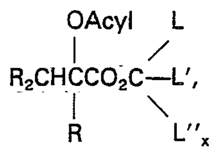 or

a hydroxy ester compound,

(B) 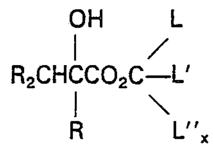

or both wherein each R is H or $C_1$ to $C_{30}$ hydrocarbyl, usually H or $C_1$ to $C_{10}$ hydrocarbyl, and each R is the same or different and L, L' and L'' are each H or an organic radical and L and L' can be members of a ring together with C, and x either is zero or 1 as necessary to satisfy the valence of the carbon atom, and wherein each of L, L', and L'' and said Acyl radicals can contain up to 30, usually up to 10, carbon atoms. Most usually each R group is limited to a maximum of 10 carbon atoms, all of which are members of an open chain hydrocarbyl group.

In a more particular aspect of the invention the enol acylate reactant is a vinyl acylate, $H_2C=CHOAcyl$, (the foregoing enol acylate where all R's are H) and compounds (A) and (B) become respectively, compounds (C) and (D):

(C) 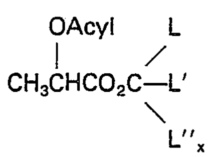

(D) 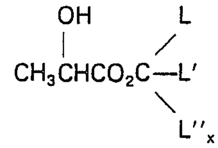

In all of the above L, L' and L'' and the residue of the Acyl attached to the Acyl O atom are usually hydrocarbyl. Moreover, the enol acylate is always free of acetylenic unsaturation and contains only one ethylenic double bond.

In another aspect of the invention, there is provided a two step process in which the product (A) and/or (B) including (C) and/or (D) is hydrolyzed to the alpha hydroxy acid:

(E) 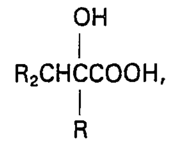 or

(F) 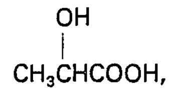 lactic acid

The process of the present invention when applied to a vinyl acylate reactant is of special importance since compounds (C) and (D) are precursors of lactic acid, compound (F), important commercially in the baking industry, in the cheese industry, in dying wool, in alkyd resins, to make plasticizers for resins, etc. It is particularly applicable when the vinyl acylate is vinyl acetate, since the latter raw material is inexpensive and readily available.

This embodiment of the invention, the last-mentioned aspect, is especially important since lactic acid has heretofore been made by relatively disadvantageous processes.

Thus, lactic acid is currently produced either by fermentation, usually of molasses, or by a multistep non-catalytic route involving HCN hydrocyanation of acetaldehyde followed by hydrolysis of the product cyanohydrin with $H_2SO_4$ to yield a stoichiometric amount of $(NH_4)_2SO_4$ along with lactic acid. The fermentation process is costly and inefficient in that it produces large amounts of by-products, making product purification expensive and troublesome. The chemical route is highly corrosive, consumes stoichiometric amounts of toxic HCN and $H_2SO_4$, is expensive, based on HCN prices, and produces stoichiometric quantities of ammonium sulfate, a low-grade fertilizer difficult to sell at a profit.

It is an object of the present invention to provide a process for making compounds of the formula (A) or (B), precursors for 2-hydroxycarboxylic acids, including compounds of formulas (C) and (D), precursors for lactic acid, by an economically advantageous route.

It is another object of the invention to provide such a process for making lactic acid precursors, and for making lactic acid therefrom by

hydrolysis, which avoids the disadvantages of the foregoing conventional processes.

Other objects, as well as aspects, features, and advantages of the present invention will become apparent from the description, examples and claims herein.

The foregoing objects are realized according to the process of the present inventions, which process utilizes inexpensive CO to replace HCN as the carbon source and is capable of producing the esters of compounds (A) and (B) in high yields at high conversions and selectivities. Furthermore, the present process results in essentially no non-recyclable or other undesirable by-products. Note that compounds (A), (B), (C) and (D) are hydrolyzable to the 2-hydroxy carboxylic acid, lactic acid in the case of (C) and (D).

We believe that we are the first to discover or disclose the carbonylation or carboxylation reaction of an enol acylate with a CO and a hydroxyl compound such as an alcohol or the like.

Thus, in U.S. Patent 4,257,973 there is disclosed in column 2 the alkoxycarbonylation of certain alcyloxyolefinic compounds in which the acyloxy group is attached to a carbon atom which is part of a hydrocarbyl group which is in turn attached to a carbon atom of an ethylenic double bond. A similar structure is disclosed in U.S. Patent 3,859,319. However, neither patent discloses the reaction of the alkoxycarbonylation of an enol acylate with CO and a hydroxyl compound, although each prior art patent discloses this type of reaction with a very wide variety of aliphatically unsaturated compounds, using particular organophosphorus palladium halides as catalysts.

U.S. Patent 4,377,708 discloses the hydrocarboxylation of enol esters such as vinyl acetate and other vinyl esters using CO and water as reactants with the enol esters. Special precautions are taken to provide for stability of catalyst, product and starting material in the presence of the water reactant, and care must be taken to provide for the presence of only a small fraction of the water required for the reaction; thus usually the water is kept at a concentration of no more than about 3 weight percent of the reaction medium, so as to avoid hydrolysis of the ester starting material such as vinyl acetate to form an enol and a carboxylic acid, which enol can and will rearrange to an aldehyde.

The present process avoids the problem of hydrolysis with water. Furthermore, as will be seen, much higher yields can be produced in the present process; the best in U.S. 4,377,708 being 76 percent selectivity at 98 percent conversion, amounting to about 74.5 percent yield, well below the yield of products hydrolyzable to lactic acid that can be made in the present process.

German patent 1,221,224 discloses carboxylation with CO and alcohols or phenols. The substrates disclosed are olefinic compounds but do not include any enol acylates. Swiss patent 589,571 is similar and also does not disclose any enol acylate starting material.

Enol acylates are potentially subject to the probability or possibility of instability in a reaction involving an alcohol or the like because some transesterification could take place to make an enol, which is unstable and would decompose to a saturated aldehyde. No such problem exists in the alkylcarboxylation of olefinic materials such as allyl acetate or methyl acrylate because no enols are potentially involved.

The hydrocarboxylation reaction is carried out catalytically, discussed in more detail hereafter. It can also be carried out continuously or in batch operation in the liquid or vapor phases. Usually the reaction is carried out in batch operation in a solvent under pressure.

The reactant concentrations can vary widely and are not critical. The ratio of hydroxyl reactant to the enol ester is usually no greater than 10/1 on a molar basis. The amount of monoxide can vary widely, but it is preferred to carry out the reaction under a carbon monoxide pressure of 15 to 3500 psig (103.4—24,132 kPag), preferably 500 to 2500 psig (3,447—17, 237 kPag). The mount of catalyst can also vary widely. Most conveniently, the amount of catalyst is between 0.01 and 100 mole percent based on the enol ester, more usually 0.1 to 10 mole percent.

Usually, the reactant is carried out with a solvent. The solvent should be inert under the reaction conditions and should dissolve the reactants and desirably dissolve the active catalyst species, although heterogenous catalysts are possible. Suitable solvents are tetrahydrofuran, benzene, $CH_3CN$, diethyl ether, diethylene glycol dimethyl ether, $CH_2Cl_2$ and $CH_3Cl$. The now preferred solvent is tetrahydrofuran, particularly when using $(\phi_3P)_2PdCl_2$ or $Pd(P\phi_3)_4$ catalyst, or other palladium compounds, although an excess of the hydroxyl compound is also especially useful. Usually, the amount of solvent in the system will be such that the enol ester concentration is at least about 0.01 weight percent in the solution. A special case of a solvent that is not inert, strictly speaking, under the reaction conditions is either of the starting material reactants, i.e., the enol ester or the hydroxy compound. Either can be used in excess of stoichiometric amount to react with the other reactant. Use of a large excess of the compound.

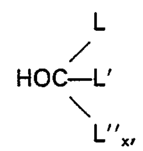

whether or not another solvent, such as THF, is present, helps produce appreciable amounts of compound (B); thus, the Acyl group is transesterified during the main carboxylation step.

The reaction is normally carried out at a temperature of 0 to 250°C, preferably 20 to 150°C. However, the reaction temperature can be below or above this if desired. As will be understood, optimum reaction temperature varies with the specific reactants. Reaction times on the order of

0.1 to 250 hours can be employed, with reaction times on the order of 2 to 50 hours being more convenient.

While a wide variety of complexes of transition metals are known as catalysts for the hydrocarboxylation of alkenes (See, for instance, 1) Pino, P., Piacenti, F., in *Organic Synthesis via Metal Carbonyls,* Volume 2, Wender, I., Pino, P., eds., Wiley New York, 1977, pp. 233—296; 2) Falbe, J., *New Syntheses with Carbon Mononoxide,* New York, Springer Verlag, Chapter 3 and 5; 3) Forster, D.,; Hershman, A.,; Morris, D. E., *Catal. Rev.—Sci. Eng. 23,* 89—105 (1981); 4) Parshall, G. W., Catal. Rev. Sci—Eng., 23, 107—124 (1981); 5) Bittler, J. V. Kutepow, N., Neubauer, D. Reis, H., *Angew. Chem. Intl. Ed. Eng.,* 7, 329—335 (1968),) no disclosures were found using such catalysts to react an enol acylate with CO and an organic hydroxyl compound. Catalysts useful are generally transition metal compounds, particularly coordination complexes of such metals. We have discovered that palladium coordination complexes are remarkably effective, and especially those complexed with a phosphine, such as $P\phi_3$, when considered in the light of experience with other known transition metal catalyst complexes for hydrocarboxylation of alkenes, or for hydroformylation for enol ethers or enol acetates (U.S. Patent 3,888,880; B. Fell, M. Barl, *J. Mol. Catal.,* 1977, 2, 301—6; Tinker, Harold B. (Monsanto) Ger. Offen 2,623,673; U.S. 4,072,709). Especially useful Pd complexes are $(\phi_3P)_2PdCl_2$ and $(\phi_3P)_4Pd$ with or without a promoter or "co-catalyst" such as HCl or $P\phi_3$.

Once a reaction is completed, the ester products can be recovered from the reaction system in a conventional manner, such as for example, by vacuum distillation.

Especially useful hydroxyl compounds in the reaction with the enol acylates and CO are hydroxyalkanes having $C_1$ to $C_4$ carbon atoms, and phenol.

The second step in the overall process of the invention is the hydrolysis of the esters (A) and/or (B) to the alpha-hydroxy acid, lactic acid in the special case of the ester products (C) and (D). This reaction is a conventional hydrolysis reaction. It is catalyzed by any dilute aqueous acid or base. Suitable acids or bases are HCl, $H_2SO_4$, $HNO_3$, $H_3PO_4$, acetic acid, KOH, NaOH, $NH_4OH$ or pyridine. The reaction is conveniently carried out at temperatures at above 0°C to 120°C, more usually about 20 to 100°C. If desired, non-interferring hydrophilic solvents other than water can be employed. Examples of such solvents are tetrahydrofuran, $CH_3CN$, and the like. Reaction times on the order of 0.1 to 24 hours, usually 0.5 to 4 hours, can be employed.

An alternate method of carrying out the hydrolysis of compounds (A) or (B) is to treat (A) or (B) with $H_2O$ in the gas phase (temperature 100—250°C) with or without catalyst.

The 2-hydroxy acid product of the hydrolysis can be recovered from the reaction medium in a conventional manner. For example, the reaction product can be recovered by precipitation as the calcium salt. Alternatively, low boiling by-product components of the hydrolysis product mixture can be removed by fractional distillation, and the remaining lactic acid or other 2-hydroxy acid solution in water can be sold as end product.

If the 2-hydroxy acid is produced by the gas phase reaction described above, it can be isolated in pure form from the reaction effluent by fractional distillation and stored either as pure compound or in aqueous solution.

It will be noted that, as understood in the art, aqueous solutions of lactic acid more concentrated than 20 percent will tend to polymerize.

The above reaction scheme comprising reactions (1) to (3) provides a simple and straightforward system for producing 2-hydroxy acids using an enol acylate starting material. As can be seen, it totally avoids the use of costly and deleterious HCN and the production of unwanted by-product ammonium sulfate, with the consumption of large amounts of sulfuric acid.

The following examples are merely illustrative and are not to be considered as limiting.

Example 1

0.48 mmoles vinyl acetate and 2.5 mmoles methanol were charged into a 71 cc stainless steel bomb equipped with a glass liner and a Teflon coated stir bar. Ten mole percent, based on vinyl acetate, of a catalyst comprising bis(triphenylphosphine) dichloropalladium, $(\phi_3P)_2PdCl_2$, was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allow to react for 44 hours with stirring. At the termination of the reaction, the reaction products were analyzed by gas chromatography and it was found that conversion was 84.5 percent and that methyl 2-acetyloxypropanoate was produced in a yield of 81.6 percent and that methyl 2-hydroxypropanoate was produced in a yield of 4 percent, based on the vinyl acetate reactant charged. Note that each of the products is hydrolyzable to lactic acid.

Example 2

0.50 mmoles vinyl acetate and 2.5 mmoles methanol were charged into a 71 cc stainless steel bomb equipped with a glass liner and a Teflon coated stir bar. Thirteen mole percent, based on vinyl acetate, of a catalyst comprising $Pd(P\phi_3)_4$ plus 10 mole percent HCl were added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb

was heated to 100°C and allowed to react for 44 hours with stirring. At the termination of the reaction, the reaction products were analyzed by gas chromatography and it was found that methyl 2-acetyloxypropanoate was produced in a yield of 77 percent and that methyl 2-hydroxypropanoate was produced in a yield of 4 percent, based on the vinyl acetate reactant charged. Note that each of the products is hydrolyzable to lactic acid. Conversion of vinyl acetate was 89.2 percent.

Example 3

0.50 mmoles vinyl acetate and 2.5 mmoles methanol were charged into a 71 cc stainless steel bomb equipped with a glass liner and a Teflon coated stir bar. Ten mole percent, based on vinyl acetate, of a catalyst comprising $RhCl_3 \cdot H_2O$ plus 10 mole percent of HI was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 40 hours with stirring. At the termination of the reaction, the reaction products were analyzed by gas chromatography and it was found that 2-acetyloxypropanoate was produced in a yield of 10 percent and that methyl 2-hydroxypropanoate was produced in a yield of 5 percent, based on the vinyl acetate reactant charged. Note that each of the products is hydrolyzable to lactic acid.

Example 4

0.50 mmoles vinyl acetate and 2.5 mmoles methanol were charged into a 71 cc stainless steel bomb equipped with a glass liner and a Teflon coated stir bar. Ten mole percent, based on vinyl acetate, of a catalyst comprising $NiI_2 \cdot 6H_2O$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 40 hours with stirring. At the termination of the reaction, the reaction products were analyzed by gas chromatography and it was found that methyl 2-acetyloxypropanoate was produced in a yield of 4.8 percent, based on the vinyl acetate reactant charged.

Example 5

0.50 mmoles vinyl acetate and 2.5 mmoles methanol were charged into a 71 cc stainless steel bomb equipped with a glass liner and a Teflon coated stir bar. Ten mole percent, based on vinyl acetate, of a catalyst comprising $(\phi_3P)_2Ni(CO)_2$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44.25 hours with stirring. At the termination of the reaction, the reaction products were analyzed by gas chromatography and it was found that methyl 2-acetyloxypropanoate was produced in a yield of 4.6 percent, based on the vinyl acetate reactant charged.

Example 6

0.50 mmoles vinyl acetate and 25.0 mmoles methanol were charged into a 71 cc stainless steel bomb equipped with a glass liner and a Teflon coated stir bar. Ten mole percent, based on vinyl acetate, of a catalyst comprising $Pd(P\phi_3)_4$ plus 10 mole percent HCl co-catalyst was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, it was found that the conversion of vinyl acetate was 100 percent. The reaction products were analyzed by gas chromatography and it was found that methyl 2-acetyloxypropanoate was produced in a yield of 69.5 percent and that methyl 2-hydroxypropanoate was produced in a yield of 24.4 percent, based on the vinyl acetate reactant charged. Note that each of the products is hydrolyzable to lactic acid. The product solution was clear, with no precipitate evident.

Example 7

5 mmoles vinyl acetate and 5 mmoles methanol were charged into a 71 cc stainless steel bomb equipped with a glass liner and a Teflon coated stir bar. One mole percent, based on vinyl acetate, of a catalyst comprising $[Rh(CO)_2Cl]_2$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 48.25 hours with stirring. At the termination of the reaction, the reaction products were analyzed by gas chromatography and it was found that methyl 2-acetyloxypropanoate was produced in a yield of 0.14 percent, based on the vinyl acetate reactant charged.

Example 8

0.50 mmoles vinyl acetate and 2.5 mmoles methanol were charged into a 71 cc stainless steel bomb equipped with a glass liner and a Teflon coated stir bar. Ten mole percent, based on vinyl

acetate, of a catalyst comprising $Pd(OAc)_2$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 40 hours with stirring. At the termination of the reaction, the reaction products were analyzed by gas chromatography and it was found that methyl 2-acetyloxypropanoate was produced in a yield of 6 percent based on the vinyl acetate reactant charged.

Example 9

0.475 mmoles vinyl acetate and 2.5 mmoles methanol were charged into a 71 cc stainless steel bomb equipped with a glass liner and a Teflon coated stir bar. 2.2 mole percent, based on vinyl acetate, of a catalyst comprising bis(triphenylphosphine)dichloropalladium $(\phi_3P)_2PdCl_2$ plus 4.2 mole percent $P\phi_3$ co-catalyst, was added. 0.05 mmoles of toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 19.5 hours with stirring. At the termination of the reaction, the reaction products were analyzed by gas chromatography and it was found that methyl 2-acetyloxypropanoate was produced in a yield of 84.3 percent and that methyl 2-hydroxypropanoate was produced in a yield of 2.7 percent, based on the vinyl acetate reactant charged. Note that each of the products is hydrolyzable to lactic acid. Vinyl acetate conversion was 95.2%.

Example 10

0.57 mmoles vinyl acetate and 5 mmoles methanol were charged into a 71 cc stainless steel bomb equipped with a glass liner and a Teflon coated stir bar. 8.7 mole percent, based on vinyl acetate, of a catalyst comprising bis(triphenylphosphine)dichloropalladium $(\phi_3P)_2PdCl_2$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, the reaction products were analyzed by gas chromatography and it was found that methyl 2-acetyloxypropanoate was produced in a yield of 79.9 percent and that methyl 2-hydroxypropanoate was produced in a yield of 12.6 percent, based on the vinyl acetate reactant charged. Note that each of the products is hydrolyzable to lactic acid, so that the yield of products useful to make lactic acid was 92.5 percent. Conversion of vinyl acetate was 100 percent.

Example 11

0.50 mmoles vinyl acetate and 2.5 mmoles methanol were charged into a 71 cc stainless steel bomb equipped with a glass liner and a Teflon coated stir bar. 16.9 mole percent, based on vinyl acetate, of a catalyst comprising $Pd(P\phi_3)_4$, was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 850 psi (5,860 kPa) (at room temperature) was charged to the bomb, and the bomb was heated to 100°C and allowed to react for 43.5 hours with stirring. At the termination of the reaction, the reaction products were analyzed by gas chromatography and it was found that methyl 2-acetyloxypropanoate was produced in a yield of 35.1 percent.

Example 12

0.486 mmoles vinyl acetate and 2.5 mmoles methanol were charged into a 71 cc stainless steel bomb equipped with a glass liner and a Teflon coated stir bar. 10.5 mole percent, based on vinyl acetate, of a catalyst comprising bis(triphenylphosphine)dichloropalladium, $(\phi_3P)_2PdCl_2$, was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, the reaction products were analyzed by gas chromatography and it was found that methyl 2-acetyloxypropanoate was produced in a yield of 68.5 percent.

Example 13

0.542 mmoles vinyl acetate and 2.74 mmoles methanol were charged into a 71 cc stainless steel bomb equipped with a glass liner and a Teflon coated stir bar. 5.2 mg. of $P\phi_3$ was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, the reaction products were analyzed by gas chromatography and it was found that no methyl 2-acetyloxypropanoate or 2-hydroxypropanoate was produced.

Example 14

0.548 mmoles vinyl acetate and 2.85 mmoles methanol were charged into a 71 cc stainless steel bomb equipped with a glass liner and a Teflon coated stir bar. No catalyst was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, the reaction products were analyzed by gas chromatography and it was found that no methyl 2-acetyloxypropanoate or methyl 2-hydroxypropanoate was produced.

Example 15

0.530 mmoles of vinyl acetate and 2.70 mmoles methanol were charged into a 71 cc stainless steel bomb equipped with a glass liner and a Teflon coated stir bar. One mole percent, based on vinyl acetate of HCl was added. 0.05 mmoles toluene was included as an internal standard. Five milliliters of tetrahydrofuran as a solvent were also included in the reaction system. The reaction mixture was charged under argon. The bomb was sealed and carbon monoxide at a pressure of 1000 psi (6,895 kPa) (at room temperature) was charged to the bomb, and the bomb was heated to 100°C and allowed to react for 44 hours with stirring. At the termination of the reaction, the reaction products were analyzed by gas chromatography and it was found that no methyl 2-acetyloxypropanoate or methyl 2-hydroxypropanoate was produced.

Example 16

1.2 grams of methyl 2-acetyloxypropanoate was hydrolyzed to lactic acid by adding it with stirring to 15 mL of 2N aqueous HCl solution. The reaction mixture was stirred at reflux for 4 hours. The reaction mixture was cooled to room temperature, and $CaCO_3$ was added slowly and carefully in small portions with stirring until the product solution was neutral to pH paper. Water was removed at high vacuum, and the white solid product was shown by NMR spectroscopy to contain 0.74 g of calcium lactate (84% yield).

Example 17

1.0 g

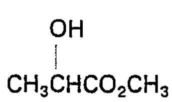

(9.6 mmol) was hydrolyzed to lactic acid by adding it with stirring to 20 mL of 2N aqueous HCl solution. The reaction mixture was stirred at reflux for 1 hour. The reaction mixture was cooled to room temperature and $CaCO_3$ was added slowly and carefully in small portions with stirring until the product solution was neutral to pH paper. Water was removed at high vacuum, and the white solid product was shown by NMR spectroscopy to contain 1.03 g of calcium lactate (98.3% yield).

**Claims**

1. A process for producing a 2-hydroxy acid which comprises;

(1) intimately admixing and reacting an enol acylate

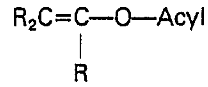

with CO and a hydroxyl compound

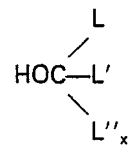

which is free of ethylenic or acetylenic unsaturation, to produce an acyloxy ester,

(A)

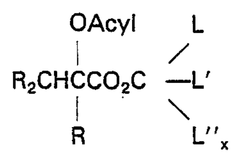

or a hydroxy ester

(B)

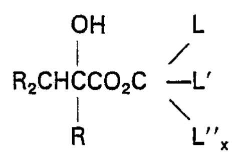

or both, wherein each R is H or $C_1$ to $C_{30}$ hydrocarbyl, usually H or $C_1$ to $C_{10}$ hydrocarbyl, and each R is the same or different, and L, L' and L'' are each H or an organic radical and L and L' can be members of a ring together with C, and x is either zero or 1 as necessary to satisfy the valence of the carbon atom, and wherein each of L, L', L'' and Acyl radicals can contain up to 30 carbon atoms, and wherein said enol acylate is free of acetylenic unsaturation and contains only one ethylenic double bond, and

(2) hydrolyzing the products of step (1) compounds (A) or (B), or both, to produce said 2-hydroxy acid,

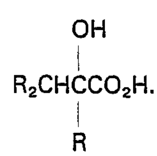

2. A process of claim 1 wherein each of L, L', L'' and said acyl radicals can contain up to 10 carbon

atoms and wherein each R group is limited to a maximum of 10 carbon atoms, all of which are members of an open chain hydrocarbyl group.

3. A process as claimed in claim 1 or claim 2 characterised in that each of L, L' and L'' and the residue of the Acyl group attached to the Acyl O atom is hydrocarbyl.

4. A process as claimed in any of claims 1 to 3 characterised for making lactic acid wherein each R is H.

5. A process as claimed in claim 1 or 4 characterised in that the hydroxyl compound reactant is $C_1$ to $C_4$ hydroxyalkane or phenol.

6. A process for making ester precursors of 2-hydroxy acids which comprises intimately admixing and reacting an enol acylate

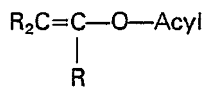

with CO and a hydroxyl compound

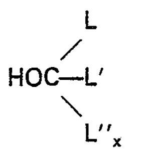

which is free of ethylenic or acetylenic unsaturation, to produce an acyloxy ester,

(A) 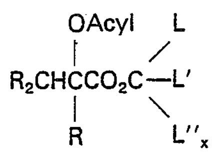

or a hydroxy ester

(B) 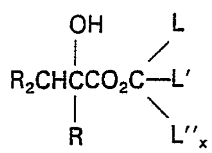

or both, wherein each R is H or $C_1$ to $C_{30}$ hydrocarbyl, usually H or $C_1$ to $C_{10}$ hydrocarbyl, and each R is the same or different, and L, L' and L'' are each H or an organic radical and L and L' can be members of ring together with C and x is either zero or 1 as necessary to satisfy the valence of the carbon atom, and wherein each of L, L', L'' and said Acyl radicals can contain up to 30 carbon atoms, and wherein said enol acylate is free of ethylenic or acetylenic unsaturation and contains only one ethylenic double bond.

7. A process as claimed in claim 6 characterised in that L, L', L'' and said acyl radicals can contain up to 10 carbon atoms and wherein each R group is limited to a maximum of 10 carbon atoms, all of which are members of an open chain hydrocarbyl group.

8. A process as claimed in claim 6 or claim 7 characterized in that each of L, L' and L'' and the residue of the Acyl group attached to the Acyl O atom is hydrocarbyl.

9. A process as claimed in claim 6 characterised for making lactic acid precursors wherein each R is H.

10. A process as claimed in claim 6 characterised in that the hydroxyl compound reactant is a $C_1$ to $C_4$ hydroxyalkane or phenol.

**Patentansprüche**

1. Verfahren zur Herstellung einer 2-Hydroxysäure, das umfaßt:

(1) ein inniges Mischen und Reaktion eines Enolacylates

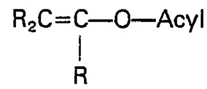

mit CO und einer Hydroxylverbindung

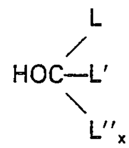

die frei ethylenischer oder acetylenischer Ungesättigtheit ist, um einen Acyloxyester

(A) 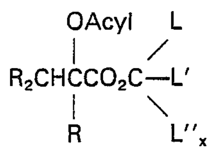

oder einen Hydroxyester

(B) 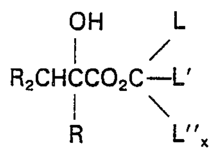

oder beide herzustellen, worin jedes R, H ist oder eine $C_1$ bis $C_{30}$ Kohlenwasserstoffgruppe, gewöhnlich H oder eine $C_1$ bis $C_{10}$ Kohlenwasserstoffgruppe, und jedes R gleich oder verschieden ist, und L, L' und L'' jedes H oder ein organisches Radikal sind und L und L' Glieder eines Rings zusammen mit C sein könnenund X entweder 0 oder 1 ist, wie dies notwendig ist, um der Wertigkeit des Kohlenstoffatoms zu genügen, und worin jedes der L, L', L'' und Acylradikale bis zu 30 Kohlenstoffatomen enthalten kann, und worin das genannte Enolacylat frei von acetylenischer Ungesättigtheit ist und nur eine ethylenische Doppelbindung enthält, und

(2) Hydrolyse der Produkt von Schritt (1), Verbindungen (A) oder (B), oder beide, um obengenannte 2-Hydroxysäure herzustellen

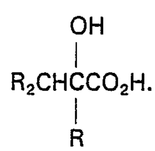

2. Verfahren nach Anspruch 1, wobei jedes der L, L', L'' und der genannten Acylradikale bis zu 10 Kohlenstoffatome enthalten kann und wobei jede R-Gruppe auf eine Höchstzahl von 10 Kohlenstoffatomen begrenzt ist, wobei alle Glieder einer offenkettigen Kohlenwasserstoffgruppe sind.

3. Verfahren nach Anspruch 1 oder 2, dadurch gekennzeichnet, daß jedes der der L, L' und L'' und der Rest der Acylgruppe, der am Acyl-O-Atom angebracht ist, eine Kohlenwasserstoffgruppe ist.

4. Verfahren nach einem oder mehreren der Ansprüche 1 bis 3, gekennzeichnet zur Herstellung von Milschsäure, wobei jedes R H ist.

5. Verfahren nach Anspruch 1 oder 4, dadurch gekennzeichnet, daß der Hydroxylverbindungs-Reaktand ein $C_1$ bis $C_4$ Hydroxyalkan oder Phenol ist.

6. Verfahren zur Herstellung von Estervorläufern von 2-Hydroxysäuren, das umfaßt inniges Mischen und Reaktion eines Enolacylats

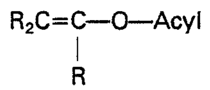

mit CO und einer Hydroxylverbindung,

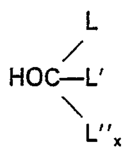

die frei von ethylenischer oder acetylenischer Ungesättigtheit ist, um einen Acyloxyester

(A) 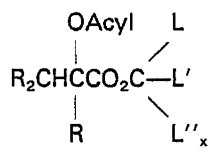

oder einen Hydroxyester

(B) 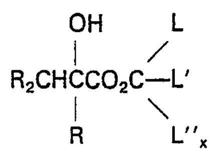

oder beide herzustellen, wobei jedes R H ist oder eine $C_1$ bis $C_{30}$ Kohlenwasserstoffgruppe, gewöhnlich H oder $C_1$ bis $C_{10}$ Kohlenwasserstoffgruppe, und jedes R gleich oder unterschiedlich ist, und L, L' und L'' jedes H oder ein organisches Radikal sind und L und L' Glieder eines Rings zusammen mit C sind und X entweder 0 oder 1 ist, wie dies notwendig ist, um der Wertigkeit des Kohlenstoffatoms zu genügen, und wobei jedes von L, L', L'' und genannten Acrylradikalen bis zu 30 Kohlenstoffatome enthalten kann, und worin genanntes Enolacylat frei von ethylenischer oder acetylenischer Verunreinigung ist und nur eine ethylenische Doppelbindung enthält.

7. Verfahren gemäß Anspruch 6, dadurch gekennzeichnet, daß L, L' und L'' und obengenannte Acylradikale bis zu 10 Kohlenstoffatome enthalten können und worin jede R-Gruppe bis zu einer Höchstzahl von 10 Kohlenstoffatomen begrenzt ist, wobei alle Glieder einer offenkettigen Kohlenwasserstoffgruppe sind.

8. Verfahren nach Anspruch 6 oder 7, dadurch gekennzeichnet, daß jedes von L, L' und L'' und der Rest der Acylgruppe, der an dem Acyl-O-Atom angebracht ist, eine Kohlenwasserstoffgruppe ist.

9. Verfahren nach Anspruch 6, gekennzeichnet durch die Herstellung von Milchsäurevorläufern, wobei jedes R H ist.

10. Verfahren nach Anspruch 6, dadurch gekennzeichnet, daß der Hydroxylverbindung-Reaktand ein $C_1$ bis $C_4$ Hydroxyalkan oder Phenol ist.

**Revendications**

1. Procédé de production d'un acide 2-hydroxylé, qui comprend:

(1) le fait de mélanger intimement et de faire réagir un acylate d'énol

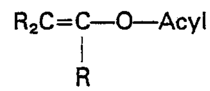

avec du CO et avec un composé hydroxylé

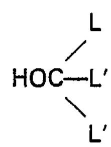

qui ne comporte pas d'insaturation éthylénique ou acétylénique, pour donner un ester acyloxylé,

(A) 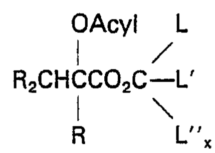

ou un ester hydroxylé

(B) 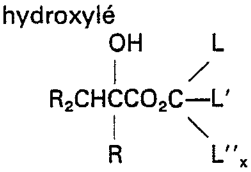

ou les deux, où chaque R est H ou un groupe hydrocarbyle $C_1$ à $C_{30}$, habituellement H ou un groupe hydrocarbyle $C_1$ à $C_{10}$, et où chaque R peut être le même ou différent; et où L, L' et L'' sont chacun H ou un radical organique et où L et L' peuvent être des éléments d'un composé cyclique formé avec C, x étant soit zéro soit 1 selon ce qui est nécessaire pour satisfaire la valence de l'atome de carbone; et où chacun de L, L', L'' et desdits radicaux acyle peut contenir jusqu'à 30 atomes de carbone, et où ledit acylate d'énol ne

comporte pas d'insaturisation acétylénique et ne contient qu'une double liaison éthylénique, et

(2) le fait d'hydrolyser les produits de l'étape (1), composés (A) ou (B), ou les deux, pour donner ledit acide 2-hydroxylé,

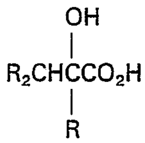

2. Procédé selon la revendication 1 dans lequel chacun de L, L', L'', et desdits radicaux acyle peut contenir jusqu'à 10 atomes de carbone et dans lequel chaque groupe R est limité à un maximum de 10 atomes de carbone, dont tous sont membres d'un groupe hydrocarbyle à chaîne ouverte.

3. Procédé selon la revendication 1 ou la revendication 2, caractérisé en ce que chacun de L, L' et L'' et le résidu du groupe acyle lié à l'atome de l'acyle est le groupe hydrocarbyle.

4. Procédé selon l'une quelconque des revendications 1 à 3, caractérisé pour fabriquer de l'acide lactique dans lequel chaque R est H.

5. Procédé selon la revendication 1 ou 4, caractérisé en ce que le réactif composé hydroxylé est une hydroxyalcane $C_1$ à $C_4$ ou un phénol.

6. Procédé de fabrication des précurseurs esters d'acides 2-hydroxylés, qui comprend le fait de mélanger intimement et de faire réagir un acylate d'énol

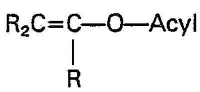

avec du CO et avec un composé hydroxylé

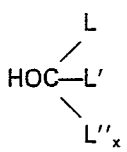

qui ne comporte pas d'insaturation éthylénique ou acétylénique, pour donner un ester acyloxylé,

(A) 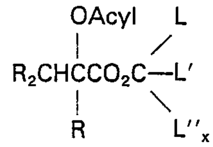

ou un ester hydroxylé

(B) 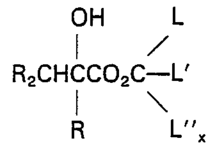

ou les deux où chaque R est H ou un groupe hydrocarbyle $C_1$ à $C_{30}$, habituellement H ou un groupe hydrocarbyle $C_1$ à $C_{10}$, et où chaque R peut être le même ou différent; et où L, L' et L'' sont chacun H ou un radical organique et où L et L' peuvent être des éléments d'un composé cyclique formé avec C, x étant soit zéro soit 1 selon ce qui est nécessaire pour satisfaire la valence de l'atome de carbone; et où chacun de L, L', L'' et desdits radicaux acyle peut contenir jusqu'à 30 atomes de carbone, et où ledit acylate d'énol ne contient pas d'insaturation éthylénique ou acétylénique et ne contient qu'une double liaison éthylénique.

7. Procédé selon la revendication 6, caractérisé en ce que L, L', L'' ainsi que lesdits radicaux acyle peuvent contenir jusqu'à 10 atomes de carbone et dans lequel chaque groupe R est limité à un maximum de 10 atomes de carbone, qui sont tous membres d'un groupe hydrocarbyle à chaîne ouverte.

8. Procédé selon la revendication 6 ou la revendication 7, caractérisé en ce que chacun de L, L' et L'', ainsi que le résidu du groupe acyle lié à l'atome 0 de l'acyle est le groupe hydrocarbyle.

9. Procédé selon la revendication 6 caractérisé pour fabriquer des précurseurs de l'acide lactique dans lesquelles chaque R est H.

10. Procédé selon la revendication 6, caractérisé en ce que le réactif composé hydroxylé est un hydroxyalcane $C_1$ à $C_4$ ou un phénol.